# EUROPEAN PATENT SPECIFICATION

(11) **EP 4 400 161 B1**
(45) Date of publication and mention of the grant of the patent: **19.02.2025**
(21) Application number: 23151429.0
(22) Date of filing: 12.01.2023
(51) Int. Cl.: A61N 5/10

(54) **QUALITY ASSURANCE SYSTEM FOR PARTICLE RADIATION THERAPY**
QUALITÄTSSICHERUNGSSYSTEM FÜR PARTIKELSTRAHLENTHERAPIE
SYSTÈME D'ASSURANCE QUALITÉ POUR RADIOTHÉRAPIE PAR PARTICULES

(43) Date of publication of application: 17.07.2024
(73) Proprietor: Terapet SA, 1242 Satigny (CH)
(72) Inventor: PALM, Marcus, 1273 Arzier-Le Muids (CH); VALLGREN, Christina, 1273 Arzier-Le Muids (CH); BRUNT, Ben, 1201 Geneva (CH)
(74) Representative: reuteler & cie SA

(56) References cited:
- EP-A1- 3 896 494
- WO-A2-2021/140233
- US-A1- 2021 299 474
- US-B2- 9 707 411
- HELMBRECHT STEPHAN ET AL: "Systematic analysis on the achievable accuracy of PT-PET through automated evaluation techniques", ZEITSCHRIFT FUR MEDIZINISCHE PHYSIK, vol. 25, no. 2, 1 June 2015 (2015-06-01), DE, pages 146 - 155, XP093054683, ISSN: 0939-3889, DOI: 10.1016/j.zemedi.2014.08.004

## Description

The present invention relates to a system for quality assurance of particle radiation therapy, in particular for measuring secondary radiation induced by particle-target interactions. The target may include but is not limited to patient, animal, phantom of various materials, cadaver, mixed or heterogeneous tissues, organs or other biological samples.

During radiation therapy, the patient is commonly irradiated by particle beams from several different angles by relative movement (translation and/or rotation) between a patient treatment couch (on which the patient is placed) and a rotating beam-guide structure (a gantry). The precision and accuracy with which an intended dose distribution can be delivered to an irradiation target in a patient primarily depends on two categories of error sources:
*(i) Target external & Mechanical factors*
   - Alignment: how well different devices are aligned to a common virtual reference point (the "isocenter"). Examples of such devices are the treatment couch on which the patient is placed, a gantry, optical guidance systems used for device calibration and quality assurance, or imaging devices used for verifying patient positioning in direct relation to the irradiation.
   - Beam delivery: errors in e.g. beam position, energy, direction, current, dwell-time per spot or shape may cause over- or under-dosage.
   - Target setup errors: differences in the positioning of the target volume with respect to the intended position or that assumed in the planning of the treatment. This may result from factors such as displacement or rotation of the target or deformation of the target.
*(ii) Target modelling factors*

Unlike photonic radiotherapy using X-rays or gamma rays, particle radiotherapy (in particular when using protons or ions) is very sensitive to range uncertainties. Unlike an exponentially decreasing depth-dose curve, the depth-dose profile of a charged particle slowing down in a material ends in a sharp Bragg peak: a very high dose is delivered just before the particle stops. This has the advantage that one may tailor the combined dose from particles or different energies delivered from one or more angles to produce an intended dose in a target region while minimizing dose to e.g. healthy tissue. At the same time, correct modelling of the slowingdown process of the particles in the target is crucial to correctly predict where the particles will stop. Modelling errors may be caused by e.g. X-ray or CT-image artefacts, anatomical changes of the patient during the treatment course, variations of how the target is set-up and immobilized on the treatment couch, or Treatment Planning System (TPS) errors in predicting stopping powers throughout various parts of the target.

Quality Assurance (QA) devices are used to assess, quantify and, as far as reasonably possible, eliminate or via calibration compensate for any clinically relevant impact of various error sources. QA procedures should be regularly carried out to provide confidence that patients receive the intended dose distribution. Examples are described below.

### Beam QA measurements

One category of QA measurements relates to those aiming to verify that relevant properties of the beam are within acceptable bounds. Such properties may be the size, shape (profile) and position of individual beams, or spots, typically measured near the isocenter in a plane orthogonal to the beam axis. Other measurements may aim to calibrate, or validate the calibration of, beam intensity monitors or dose monitors, which are used to determine when the assigned number of particles have been delivered to a smallest subset of a treatment plan (a "spot"). Such measurements are critical to ensure that an acceptable dose distribution is delivered, and typically require sub-millimeter and sub-percent precision and accuracy for spatial measurements and charge measurements, respectively.

### QA devices based on destructive ionization measurements

US2010/0108901 discloses a multi-layer ionization chamber intended for measuring a 1-dimensional depth-dose profile of a proton beam by intercepting, absorbing and stopping the beam. The device can be placed on the treatment couch, must face the beam, and can measure the depth-dose curve of single proton beam spots ("Bragg curve") or the combined depth-dose curve from several beam spots that may form a Spread-Out Bragg Peak. This type of prior art has the drawback that the measurement is destructive: it is not possible to noninvasively measure the dose distribution in a target. It is possible to indirectly measure total beam energy loss in a target by shooting a proton beam through the target and measure a shift in a Bragg curve as compared to shooting a proton beam of the same initial energy directly on the multi-layer ionization chamber. However, with this method, it is not possible to probe internal variations of the beam stopping power along the beam axis inside the target: only an integral stopping power across the target can be measured by the device. A depth-dose measuring device could be used to produce a tomographic stopping-power image of a target only by repeated measurements of integral stopping power from multiple directions ("proton tomography"). It is a drawback that this would require a large series of measurements, combined with repeated repositioning of the target or the device setup, since the device can only intercept the beam from a single direction (perpendicularly to the ionization chambers).

The device disclosed in US2010/0108901 cannot intercept proton beams delivered from many different directions as requested in typical clinically relevant treatment plans. In addition, the device also has a limited aperture of a few cm diameter within which the beam may be intercepted. This is smaller than the available cross section of a scanned beam field, which may span 20 x 20 cm, or larger, depending on the beam delivery system (BDS).

Another type of QA device is disclosed in EP1907062B1, based on a plurality of smaller ionization chambers arranged in a plane. Such and similar devices may be used to verify that the transverse coordinates of a scanned beam are as intended in e.g. a plane encompassing the isocenter. But this type of device also cannot provide a direct measurement of how or where a beam is slowing down inside a target.

### Proton Tomography

An example of a device specifically intended for clinical proton tomography on patients is disclosed in US2011220794A1. Disadvantages of this device include:
- the target must be irradiated from many different directions by rotating the detection elements of the device in unison with a rotation of a gantry, whereby either the device needs to be mechanically mounted on the gantry, to ensure that the device rotates in unison with the gantry, or, alternatively, the device must be integrated with the gantry rotation control system, ensuring that the device and gantry motion is coordinated,
- the available proton beam energy must be sufficiently high that the beam can fully traverse the entire target and enter the detection elements downstream of the target, which may, due to multiple Coulomb scattering in target and air gaps, significantly increase the transverse spot size of the proton beam as it reaches the target-downstream detector, effectively limiting the spatial resolution of the tomographic stopping power image,
- it is not possible to probe the stopping power in internal regions of the target without acquiring a full tomographic data set from around the target.

### Constancy check

A "constancy check" is a measurement carried out to verify that a reference treatment plan is delivered consistently after e.g. a hard- or software upgrade or machine maintenance, or as a part of a regular routine control. Typically, this is done by delivering treatment plans under welldefined conditions and ensuring that the readings from various QA-devices are consistent with earlier measurements. A disadvantage of existing QA devices is that such measurements can only be taken with a limited set of beam parameters. In particular, the QA devices in the referenced prior art do not allow for irradiation from any direction, but are restricted to "faceon" irradiation. Verification that e.g. the position and range of a scanned beam is as intended from a wide, or even continuous, range of gantry angles is challenging and impractical. Such a QA-measurement would be of particular relevance for arc therapy, consisting of continuous, or quasi-continuous delivery of a beam as the gantry rotates and/or the treatment table is moved.

### Patient-specific QA

Patient-specific QA may involve delivering a patient-specific treatment plan into a target. After delivery, log-files from devices such as beam monitors inside the radiation emitter head that may contain information relating to the actually delivered number of particles per spot, or measured positions of the scanned beam, may be used to re-calculate what the delivered dose distribution would have looked like in a patient, based on the same patient-specific model that was used to design the treatment plan. The patient-specific QA may also involve delivering the patient-specific treatment plan into e.g. a water tank with point- or 2D-ionization chambers. The measured doses are compared to a re-calculated dose distribution in water. Such QA procedures only address uncertainties in the delivery of the beam - not potential errors of the anatomical model of the patient / target that could result in e.g. over- or undershooting.

More generally, it is important to ensure that the delivered radiation is accurate both spatially and in dosage, accurately covering the intended treatment region taking into account the errors and uncertainties that occur in the chain of steps between the intended radiation treatment and the delivered radiation treatment, this chain comprising the imaging of the region of interest, the modelling of the tissue to be treated and surrounding the region of interest, the modelling of the radiation beam, the treatment plan, the set up and positioning of the radiation treatment device and finally the actual spatiotemporal delivery of the radiation beam.

As previously described, various quality assurance systems are used to control and to calibrate the radiation treatment taking into account certain of the afore mentioned errors and uncertainties. Certain quality assurance devices, at least in the literature, are intended to measure the radiation beam during treatment, and others are used principally in a factory or pre-treatment setting for calibration of radiation treatment devices prior to treatment, however such devices typically do not take into account actual treatment settings such as the radiation treatment machine and treatment table and gantry parts. The actual treatment setting however may influence the radiation beam therapy and measurements thereof.

The installation and set-up of the quality assurance system in conventional systems can be complex and time consuming which is not only costly but also makes it difficult to implement the quality assurance (QA) system during treatment or just prior to treatment. Likewise, carrying out constancy check measurements for QA-purposes or assessing the correctness of a Treatment Planning System target modelling from many different beam directions is timeconsuming, requiring use of several different measurement devices and many manual steps, in particular when using third party QA devices that are not mechanically integrated in the irradiation room, nor with the treatment apparatus control systems.

EP 3896494 discloses a gamma ray detection system for an ion beam emission apparatus and WO 2021140233 discloses an ion beam emission apparatus for radiation therapy comprising a detector apparatus movably mounted with respect to a patient treatment table.

One of the problems the invention seeks to solve is to be able to conveniently setup a QA system in an irradiation room and deliver proton beams from virtually any angle, without any modifications of existing irradiation room equipment

In view of the foregoing, it is an object of this invention to provide a quality assurance system for particle radiation therapy that is quick and easy to implement yet ensures accurate calibration or validation of particle radiation therapy to identify or reduce errors and uncertainties between the intended dose distribution and the delivered dose distribution.

It is advantageous to provide a quality assurance system for particle radiation therapy that is flexible and may be easily adapted to various beam delivery configurations and treatment tables.

It is advantageous to provide a quality assurance system for particle radiation therapy that is easy and economical to install and uninstall in an irradiation room, in particular with respect to conventional particle radiation therapy devices.

It is advantageous to provide a detection system for a quality assurance system for particle radiation therapy that is compact, easy and quick to install and uninstall, and yet that allows the accurate measurement of target activation in a representative treatment setting.

Objects of this invention have been achieved by providing a system according to the independent claims. Dependent claims set forth various advantageous features of embodiments of the invention.

Disclosed herein is a quality assurance system for radiation therapy for use in a treatment apparatus including a treatment table and a particle radiation device having a radiation emitter head for particle beam radiation, comprising
- a detector apparatus including at least one detection module configured for the measurement of gamma radiation emitted by a target object subject to a particle radiation beam, and
- a support mechanism on which the at least one detection module is mounted, the support mechanism comprising a base configured for removable coupling to a treatment table of the treatment apparatus, and at least one detection module support structure pivotally coupled to the base via a pivot coupling.

In an advantageous embodiment, the detection module support structure has a substantially C-shape with an open section configured for transmission of a particle radiation beam emitted by a radiation emitter of the particle radiation device through the open section.

In an embodiment, the detection module support structure has at least two open sections configured for transmission of a particle radiation beam emitted by a radiation emitter of the particle radiation device through the open sections.

In an advantageous embodiment, the support mechanism comprises a support arm coupling the detection module support structure to the pivot coupling and positioning the detection module support structure relative to the pivot coupling such that a center axis of the support structure is aligned with the axis of rotation of the pivot coupling.

In an advantageous embodiment, the pivot coupling comprises a motor configured for motorized rotation of the detection modules about an axis of rotation of the pivot coupling.

In an advantageous embodiment, the support mechanism of the detector apparatus further comprises a slide coupling configured for translation of a pivot base of the pivot coupling relative to the base, the slide coupling or instance comprising rails on the base engaging complementary rails or channels on the pivot base.

According to the invention, the system further comprises a detector transport apparatus including a trolley having a support surface for removably mounting the detector apparatus thereon, the trolley having ground engaging members, for instance in the form of wheels or rollers, allowing displacement of the trolley to and away from a treatment apparatus configured for positioning the detector apparatus for coupling to the treatment apparatus.

In an advantageous embodiment, the trolley of the detector transport apparatus comprises a position adjustment mechanism for moving the detector apparatus on or off the support surface of the trolley.

In an advantageous embodiment, the system further comprises a lift mechanism for transferring the detector apparatus from the trolley onto the treatment table.

In an advantageous embodiment, the system further includes an adaptor interface mountable on a treatment table of a treatment apparatus, the detector apparatus mountable on the adaptor interface for coupling to the treatment table.

In an advantageous embodiment, the adaptor interface and trolley have interengaging fixing elements for storing the adaptor interface on the trolley when it is not in use.

In an advantageous embodiment, the trolley comprises guard rails receiving ends of the detection module support structure therein for protection of the detection modules when the detector apparatus is mounted and stowed away on the detector transport apparatus.

In an advantageous embodiment, the system further includes a phantom device including a body consisting of or comprising a material selected from any one or more of polyethylene (PE), HE Solid Water, Virtual Water, RW1, RW3, polystyrene, Blue Water or Polymethylmethacrylate (PMMA), or other low-Z materials with generally short-lived radioactive isotopes produced by the beam, or isotopes with a high probability of emitting positrons after interaction with a radiation beam such as such as 18O, 63Cu or 68Zn, and one or more cavities formed within the body.

In an advantageous embodiment, the cavities of the phantom device include bores formed into the material of the body and/or slots which may include transverse slots and axial slots.

In an advantageous embodiment, the phantom device comprises a front face that is substantially planar, and a side face substantially orthogonal to the top or front face and connected to the top or front face via a chamfered or rounded corner face.

In an advantageous embodiment, the side face is substantially cylindrical or polygonal.

In an advantageous embodiment, the system further comprises a computing system connectable to the detector apparatus for acquisition of measurement data from the detection modules and for control of the detector apparatus.

In an advantageous embodiment, the computing system is configured to output at least one distal fall-off coordinate of the measured activation, the distal fall-off coordinate corresponding to a falling edge of the measured activation along the particle beam axis.

In an advantageous embodiment, the computing system is configured to output the coordinates of at least one rising edge of the measured activation, the rising edge coordinates corresponding to an entrance point of the particle beam in the irradiated target.

Also disclosed herein is a treatment apparatus in combination with a quality assurance system for radiation therapy according to any preceding embodiment, wherein in use the detector apparatus is mounted on a treatment table of the treatment apparatus and a radiation emitter head of the treatment apparatus is positioned for transmission of a particle radiation beam through an opening in the detection module support structure, the position of the detection module support structure adjusted for alignment with the radiation emitter head through the opening in the support structure.

In an advantageous embodiment, the detection module support structure is positioned within a tunnel of the treatment apparatus within which the radiation emitter head is mounted.

In an advantageous embodiment, the detection module support structure is dynamically coupled to the base and configured to rotate in a synchronized manner with the radiation emitter head of the treatment apparatus.

In an advantageous embodiment, the computing system of the quality assurance system for radiation therapy is connected to at least one of a treatment planning system module of the treatment apparatus and a beam delivery system for receiving information on the target, the treatment plan, the irradiation progress and log files from the irradiation session.

Further objects and advantageous features of the invention will be apparent from the claims, from the detailed description, and annexed drawings, in which:
Figure 1 is a block diagram of a quality assurance system for radiation therapy according to an embodiment of the invention;
Figure 2a is a schematic representation of a treatment apparatus, showing an accelerator, magnetic elements of a rotating gantry, a radiation emitter head including monitors, a scanned particle beam and a phantom device on a treatment table;
Figure 2b is a perspective illustration of a detector apparatus of a quality assurance system for radiation therapy according to an embodiment of the invention, installed in a conventional treatment apparatus including a treatment table and a particle radiation device;
Figure 3a is a perspective view of a detector apparatus of a quality assurance system for radiation therapy according to an embodiment of the invention;
Figure 3b is a view similar to figure 3a showing detection modules and support structure of the detector apparatus in a rotated and translated position relative to a base, compared to the position in figure 3a;
Figure 3c is a schematic representation of the detector apparatus seen in the direction of the rotation axis of the detector modules;
Figure 3d is a perspective view of a detector apparatus with two detection modules, the support structure being configured to have two open sections between the detector modules according to an embodiment of the invention
Figures 4a and 4b are perspective views of a detector transport apparatus of a quality assurance system for radiation therapy according to an embodiment of the invention;
Figure 5a is a perspective view of the detector apparatus of figures 3a to 3c mounted on the detector transport apparatus of figures 4a and 4b and a treatment table onto which the detector apparatus is being positioned;
Figure 5b is a perspective view of the treatment table and an adaptor interface of the detector apparatus mounted thereon according to an embodiment of the invention;
Figure 5c is an exploded perspective view of the detector apparatus being mounted on a treatment table, with an adaptor interface in between according to an embodiment of the invention;
Figure 6a is a perspective view of a phantom device of a quality assurance system for radiation therapy according to an embodiment of the invention;
Figure 6b is a simplified schematic representation of a phantom device and an associated graph illustrating the intensity of activation over the length of the phantom device with cavities formed therein, and corresponding rising edge and fall-off points along the beam trajectory;
Figure 7a is a block flow diagram illustrating steps for acquiring measurement data in a quality assurance system for radiation therapy according to an embodiment of the invention;
Figure 7b is a simplified block flow diagram illustrating steps for checking the constancy of measurements performed by a quality assurance system for radiation therapy according to an embodiment of the invention;
Figure 8 is a block flow diagram similar to figure 7a further showing steps of analysis and output in a quality assurance system for radiation therapy according to an embodiment of the invention;
Figure 9 is a block flow diagram illustrating an example of steps processing a measurement of a penetration depth of a particle beam in a target object, according to an embodiment of the invention;
Figure 10 is an illustration similar to figure 9 of a variant.

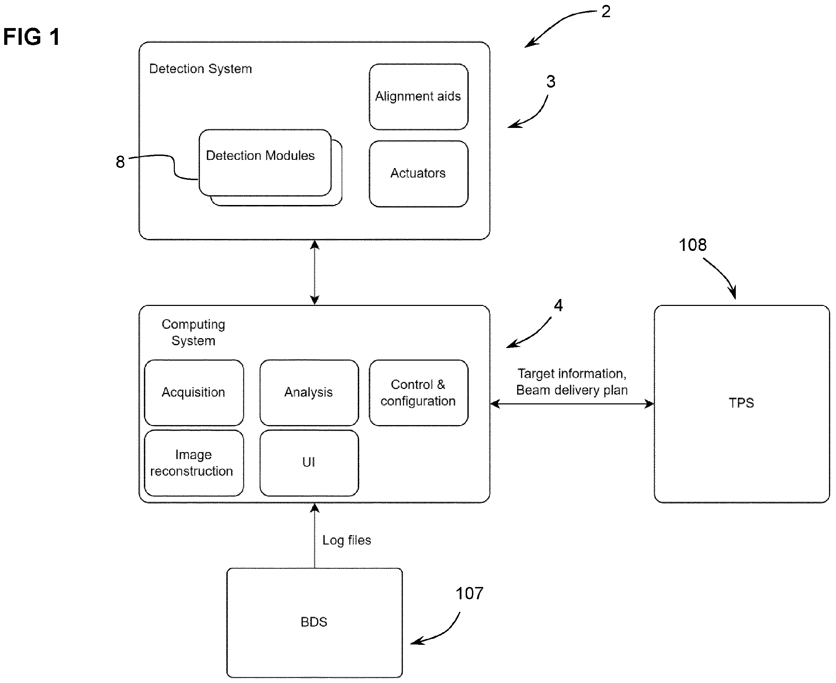

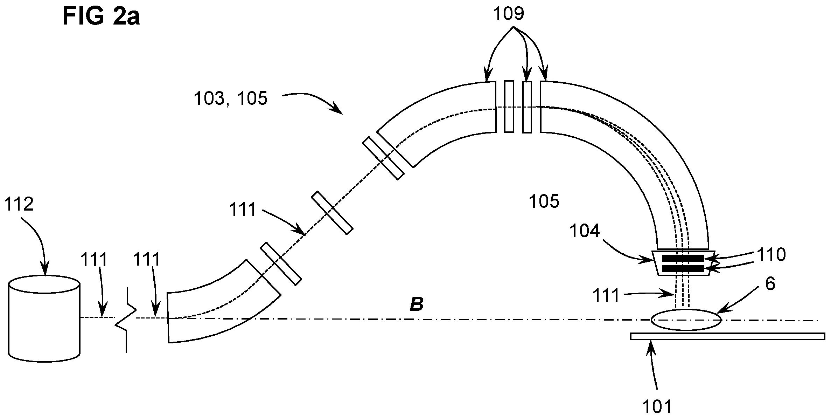

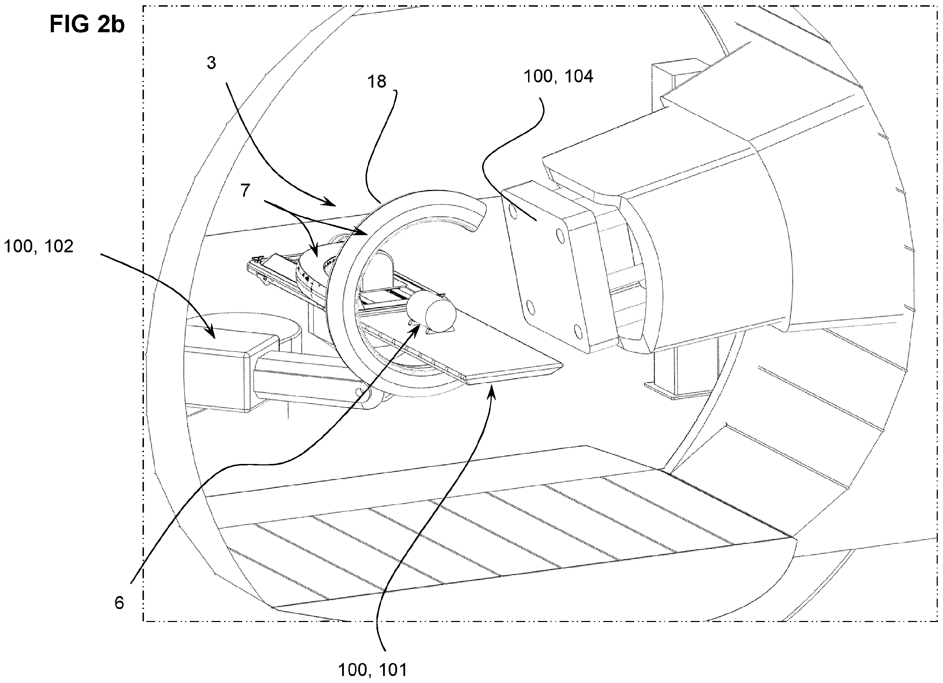

Referring to the figures, starting with figures 1 and 2, a quality assurance system for radiation therapy 2 according to an embodiment of the invention for use with a treatment apparatus 100 is illustrated. The quality assurance system for radiation therapy 2 comprises a detector apparatus 3 including detection modules 8, and a computing system 4 connectable to the detection modules for the acquisition and analysis of measurement data outputted by the detection modules.

The computing system 4 may further comprise a control module for control and configuration of the detector apparatus and data acquisition process. The computing system may yet further comprise a user interface and a module for image reconstruction to visualize relevant quantities, such as the treatment dosage and distribution, activation or stopping power, in a target body.

The computing system may further include or be connected to, and receive and transmit data with, a database, Beam Delivery System (BDS) 107, and a treatment planning system (TPS) 108 comprising information on the target to be treated and the radiation beam delivery plan. The TPS includes software used to generate a treatment plan, which may typically comprise a list of proton beam spots to be delivered based on available target information. The TPS is separate from the Beam delivery system and Control Systems that control the irradiation. The computing system may thus be connected to a treatment planning system that may be used for providing information on the target and generating a treatment plan, and a beam delivery system of the treatment apparatus 100 executing and monitoring the treatment plan delivery process that includes the control of the particle radiation device 103 for delivery of radiation treatment to a patient or target.

The treatment apparatus may have various configurations according to *per se* known treatment apparatuses. In the illustrated example, the treatment apparatus comprises a treatment table 101, also known as a patient treatment couch, which may be mounted on a robotic arm 102, and a particle radiation device 103 comprising a particle accelerator 112, a gantry 105 with beam guiding, focusing, and deflecting magnets 109 and a radiation emitter head 104 comprising beam monitors 110.

In variants, the treatment table 101 may via the robotic arm 102 have up to six degrees of freedom: translation in three dimensions and rotations (pitch, roll, yaw), independently from the gantry 105, thereby permitting irradiation from a range of directions on a patient or target placed on the treatment table 101 by adjusting the position and orientation of the treatment table 101 and the angle of the gantry around its rotation axis ***B*.** In another variant, the radiation device 103 does not comprise a gantry, the radiation emitter head 104 being fixed and providing e.g. a horizontal or vertical beam into an irradiation room. Other combinations of relative movement are possible, for instance having the accelerator 112 mounted directly on a rotating structure.

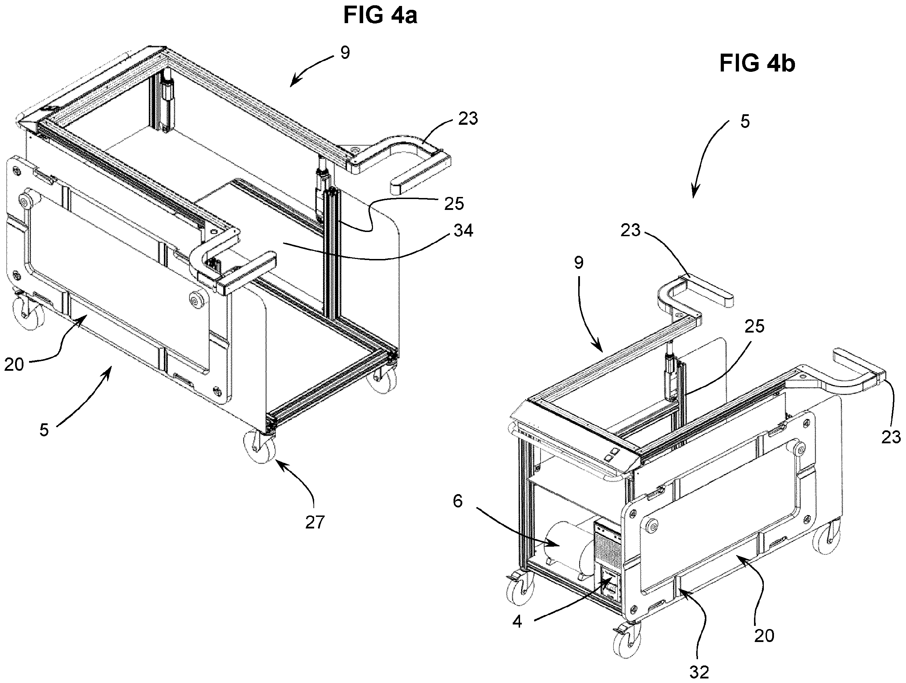

Referring more particularly to figures 2a to 5b, the quality assurance system for radiation therapy 2 according to the invention further includes a detector transport apparatus 5 for moving and placing the detector apparatus 3 into and out of a treatment setting, for instance into and out of the treatment apparatus 100 or irradiation room.

The detector transport apparatus 5 comprises a trolley 9 mounted on wheels 27 comprising an upper support surface or rails or other support elements on which the detector apparatus may be mounted when it is not installed in the treatment apparatus 100.

The quality assurance system for radiation therapy 2 according to embodiments of the invention may advantageously further include a phantom device 6 that may be used to perform measurements and acquire data from the particle radiation beam emitted by the particle radiation device 103 of the treatment apparatus 100. The phantom device is made of a material and comprises features that are intended to simulate a body region of a patient and/or provide a specific target reference body that allows *inter alia* estimation of the behavior of the particle radiation beam in a body part of a living subject during treatment.

The detector apparatus 3 comprises one or more detection modules 8 mounted on a support mechanism 7.

The detection modules 8 are configured for measurement of secondary radiation emitted by the target subject to particle radiation, the secondary radiation principally in the form of gamma rays.

Gamma ray detection modules are *per se* known and in the present invention may advantageously comprise a configuration and features as described in WO 2021140233. Other gamma ray detection modules *per se* known in the art may also be used within the scope of this invention.

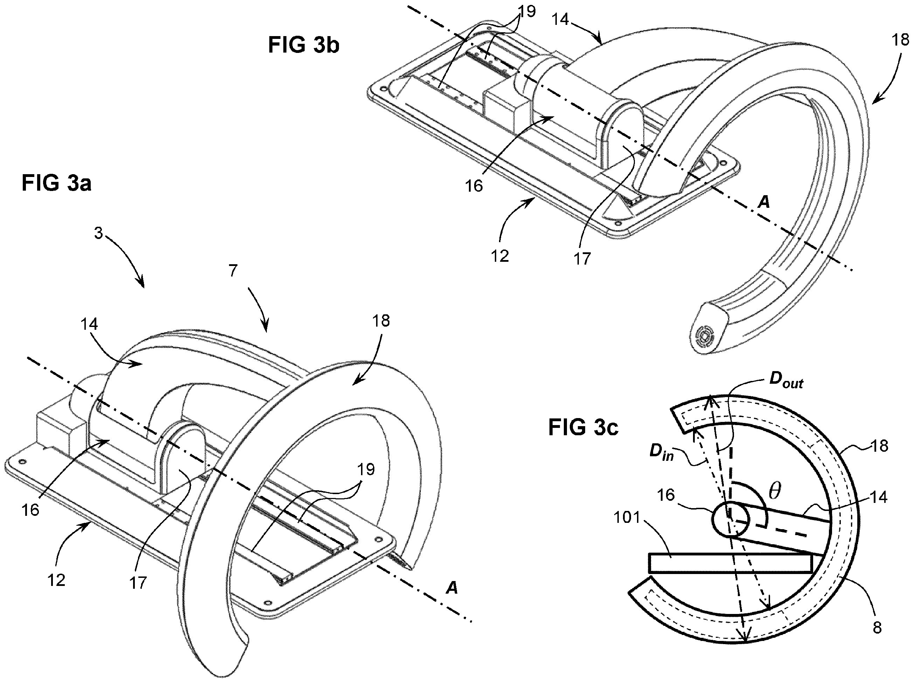

In an embodiment, the detection modules 8 are advantageously arranged in a ring around a center axis. In a preferred embodiment, as illustrated in figures 3a to 3c, the ring has an opening such that it forms a substantially C-shape, the opening allowing the radiation emitter head 104 of the particle radiation device 103 to emit the particle radiation beam 111 through the opening.

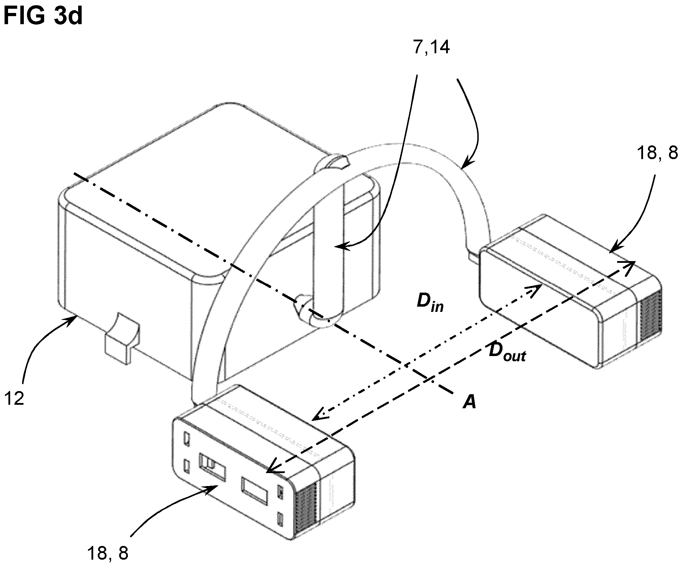

In another embodiment, the detection modules may be arranged to provide openings allowing the radiation emitter head 104 of the particle radiation device 103 to emit the particle radiation beam 111 through the openings, on opposed sides. For instance, in the embodiment illustrated in figure 3d, at least one pair of detection modules 8 are positioned on the support mechanism 7 in a spaced apart opposed manner, leaving openings on opposed sides (top and bottom sides in the illustration).

In a treatment plan that includes the relative rotation of the radiation head about the center axis, the detection module support structure 18 is configured to rotate in a manner to align the opening of the ring with the particle radiation beam position, in other words with the position of the radiation emitter head 104.

In a treatment apparatus 100 comprising a tunnel surrounding the treatment table 101, around which the radiation emitter head 104 may move, the outer diameter ***D**ₒᵤₜ* of the detection modules support structure 18 within which the detection modules 8 are mounted is configured to fit within the tunnel of the treatment apparatus 100.

The inner diameter ***Dᵢₙ***, of the detection modules support structure 18 may advantageously be configured to surround the treatment table 101 which may be received within the inner diameter of the detection modules support structure.

The support mechanism 7 of the detector apparatus comprises a base 12 upon which a support arm 14 is movably coupled, the detection module support structure 18 being fixed to the support arm 14. The support arm 14 may advantageously be coupled to the base via a pivot coupling 16 having a center of rotation that corresponds to a center axis ***A*** of the detection module support structure 18 such that as the support arm 14 is pivoted about the pivot coupling 16, the detection module support structure 18 performs a circular movement within its own envelope. The support mechanism 7 may advantageously further comprise a slide coupling 19, for instance including rails mounted on the base 12 configured for translation of a pivot base 17 of the pivot coupling 16 in the axial direction ***A*.** The detection modules 8 may thus be rotated in a circular manner about the center axis ***A*** and additionally may be translated in the direction of the center axis. The translation permits the axial positioning of the detection modules precisely over the target region and moreover if the radiation emitter head 104 or treatment table 101 is translatable in the axial direction relative to the center axis ***A*** also allows the detection modules 8 to translate in a corresponding manner. The translation also facilitates access or unobstructed view of the target if necessary, for example if the target is to be imaged with orthogonal X-ray panels *in-situ.*

The radiation emitter head, or elements on the radiation emitter head such as range shifters, may furthermore be translatable in the beam direction to reduce the gap between said elements and the target. The translation advantageously allows beam irradiation with minimal gaps by clearing said gap from the detection modules support structure 18 and support arm 14.

The pivot coupling 16 preferably comprises a motor for motorized actuation of the rotation of the detection modules 8. The slide coupling 19 may also comprise a motorized device, for instance a linear nut and screw actuator for translation of the detection modules 8 relative to the base 12.

In variants, the pivot coupling and/or slide coupling may also be entirely mechanical and manually actionable, in particular for use with a particle radiation device 103 that has a radiation emitter head 104 that is not moving dynamically during particle beam radiation.

In variants, multiple detection modules may be independently moveable, for example by means of independently pivoting support structures or by means of multiple articulated positioning arms.

The detector apparatus advantageously comprises a support mechanism 7 with a base 12 that enables the detector apparatus to be coupled to, and in particular for instance mounted on, a treatment table 101 in a treatment setting, namely within the treatment apparatus 100 in a position corresponding to a position for treatment of a patient.

The quality assurance system for radiation therapy may advantageously further include an adaptor interface 20 forming an interface between the base 12 of the detector apparatus 3 and the treatment table 101, the adaptor interface 20 being configured to adapt the position of the base 12 relative to the adaptor interface 20 via base positioning elements 32. The adaptor interface may comprise fixing members 35 for fixing the adaptor interface to the treatment table 101, whereby different fixing members for treatment tables with different standards may be provided, such that the detector apparatus 3 can be coupled to different treatment tables 101 without modification of the base 12.

In the illustrated example, the treatment table 101 comprises position markers or fixation elements 106 on a side edge thereof that allow the positioning and registration of the patient and/or the adaptor interface 20 relative to the particle radiation device 103 and/or robotic arm 102.

The trolley 9 may advantageously comprise elements for fixing and stowing away the adaptor interface 20 on the trolley, for instance on a side of the trolley for convenience.

The trolley 9 may include a cabinet chamber 34 for storing various components of the quality assurance system for radiation therapy 2 and may further support a computing system 4 and have a space for storing one or more phantom devices 6. The trolley may also support a power supply such as a battery, or provide plugs and/or cables for connecting to a power supply, for instance for connection to the detector apparatus 3.

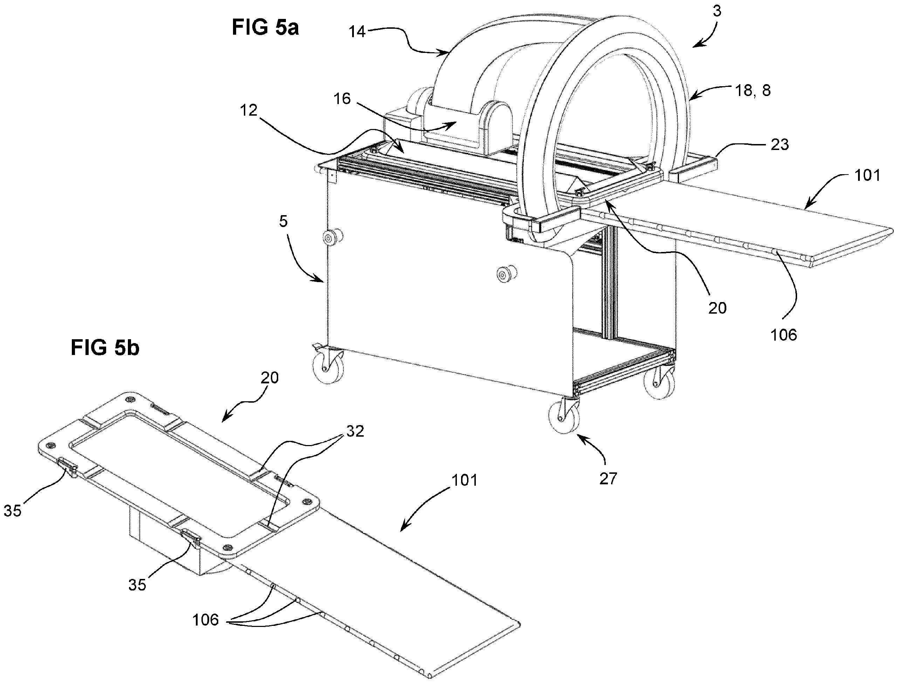

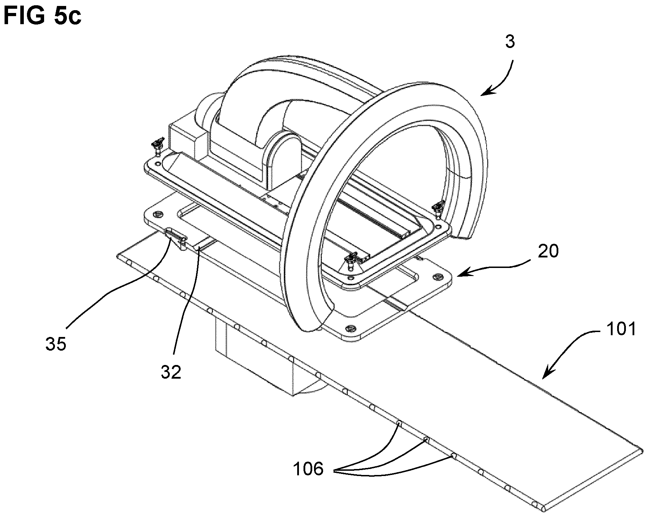

The trolley 9 may further comprise a displacement mechanism 25 including for instance a height adjustment mechanism and optionally a translation mechanism for moving the detector apparatus 3 of the trolley 9 off or onto the treatment table 101. Prior to such operation, the adaptor interface 20 may be mounted on the treatment table 101 as illustrated in figure 5b.

The trolley 9 may further comprise a motorized or manually actionable lift mechanism (height adjustment mechanism) for lifting the detector apparatus 3 off the trolley onto the treatment table 101. Alternatively, the treatment table 101 may have a lift mechanism to adjust the height of the treatment table, and which may also be used to lift the detector apparatus 3 off the trolley onto the treatment table 101. In a variant, both the treatment table and the trolley may each have lift mechanisms.

When the detector apparatus 3 is mounted on the trolley 9 for storage or when not in use, the detector modules support structure 18 may be positioned in a stow-away position as illustrated in figure 5a, with the open ends of the detection module support structure 18 positioned within guard rails 23 that provide protection to the support structure on the trolley.

The phantom device 6 according to an advantageous embodiment of the invention comprises a body 22 of a material preferably of a low average atomic number, and a plurality of cavities 24 that may include bores 26 and/or slots 28 formed into the body of the material. The material of the phantom device 6 may include any one or more of commonly used phantom materials such as polyethylene (PE), HE Solid Water, Virtual Water, RW1, RW3, polystyrene, Plastic Water, Blue Water or Polymethylmethacrylate (PMMA), or other materials with generally short-lived radioactive isotopes produced by the beam. The phantom device may also include any one or more of materials with a high concentration of isotopes with a high probability of emitting positrons after interaction with the proton beam, such as ¹⁸O, ⁶³Cu or ⁶⁸Zn. The slots 28 may include transverse slots 28a that are arranged transverse to the axial direction ***A*** and axial slots 28b, or slots that have both an axial and a transverse component (not illustrated). The body 22 may have a top or front face 30, a side face 31 which for instance may have a generally cylindrical shape although other non-axisymmetric or polygonal shapes may also be provided. The front or top face 30 may be joined to the side face 31 via a rounded or chamfered corner face 33. Both the front face and chamfered or rounded corner face may be provided with cavities that are formed into the body of the material.

The cavities 24 form regions in which the particle radiation beam has negligible loss of energy and may thus serve as spatial reference zones in the measurement of the secondary radiation resulting from interaction between the particle radiation beam and the material of the body 22 and may be configured in varying sizes, and geometrical arrangements and densities to adjust the properties of the phantom device on the particle radiation beams passing therethrough. In variants, it is possible to have a phantom device with different material structures, comprising materials with different compositions and/or densities that are formed together in layers or other 3-dimensional structures, for instance formed by additive manufacturing such as 3-D printing, to more closely simulate a target object, for instance a body part containing a treatment target region.

The diameter or width of the features of the phantom device 6 may thus be advantageously selected so as to give the greatest sensitivity to displacements of the beam from the intended position, or to relative displacements between two or more irradiation sessions under comparison. This may be achieved for example by selecting the diameter of the bores such that the edges of the bores are aligned with the greatest gradient in the transverse profile of the beam. For a Gaussian beam profile, this corresponds to a width or diameter of 2σ. The width of the beam may depend on the energy of the beam. For this reason, the phantom may advantageously feature bores with a range of diameters in order that the sensitivity to displacements may be maximized by selecting an appropriate bore for the beam energy of interest.

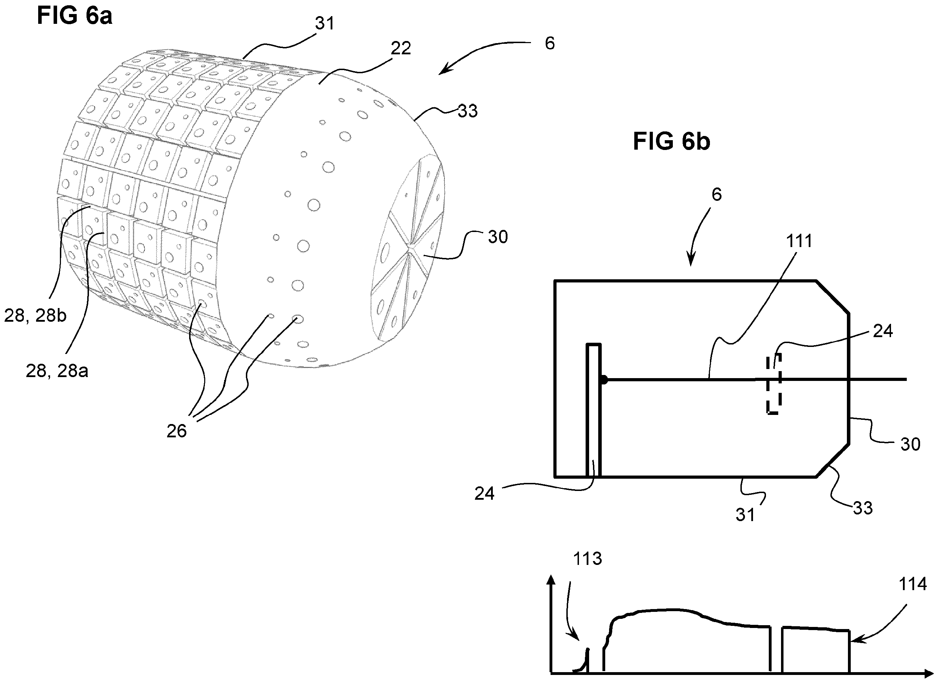

The phantom device 6 may also incorporate internal cavities 24 as schematically illustrated in figure 6b.

The material near bores, slots or cavities, may advantageously be rich on one or more isotopes chosen to increase the available signal for imaging.

The cavities may advantageously be used to enhance the sensitivity to displacements of the beam 111 along its axis, i.e. deviations of beam penetration depth. The energy of the beam may be selected such that its distal falloff region coincides with the proximal or distal surface of a cavity or recess. In a preferred embodiment, the largest gradient of the intended distal falloff coincides with the proximal or distal cavity surface. A change in beam energy could be measured as an increase or decrease of total activation detected downstream of a cavity or recess, as illustrated in Figure 6b.

It is advantageous to define the precise dimensions and material of the phantom device 6, and intended beam trajectories, such that the intended beam ranges match any of those beam energies available for clinical or quality assurance purposes. In a preferred embodiment, the surface at the beam entrance point and the distal and proximal bounding surfaces of the cavities or recesses along the beam trajectory are preferably all parallel to each other and orthogonal to the beam axis in order to avoid that the target activation up- or downstream of any cavities or recesses is also correlated to minor unintended beam position displacements.

The phantom device 6 may be reproducibly placed on the treatment table using a docking structure similar to the adaptor interface 20 that may be conveniently aligned to the treatment table position markers 106, thereby suppressing phantom device positioning uncertainty with respect to the treatment table. In an embodiment of the invention, the adaptor interface 20 is extended from the base 12 along the treatment table 101 to also serve as a docking structure or support for a target.

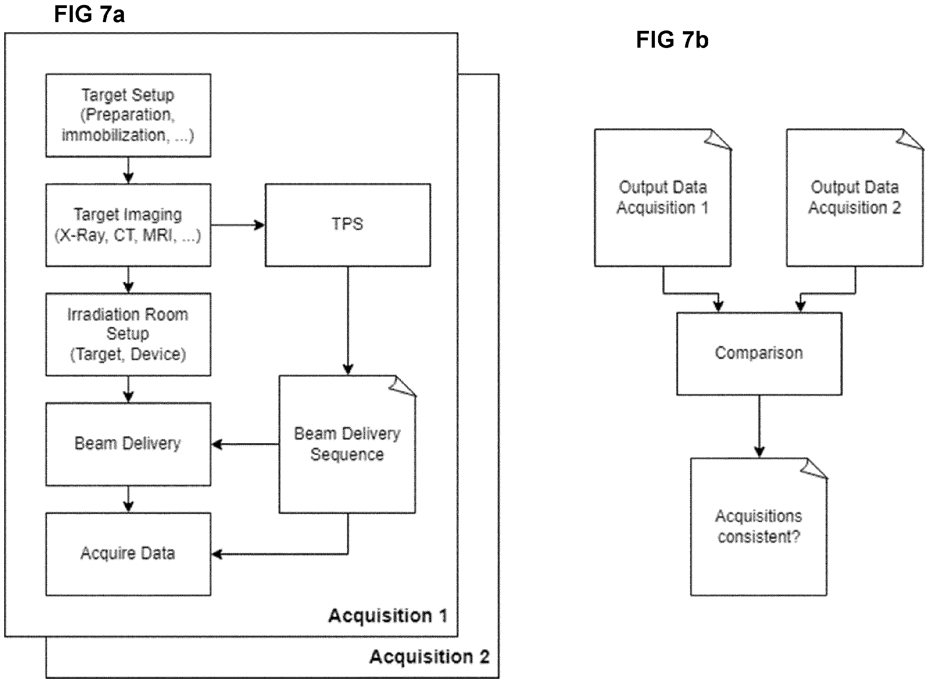

A method for performing a constancy check using the system of the invention is illustrated in Figure 7a. This method uses two or more independent sessions of irradiation, performed for example routinely to compare against a reference acquisition, or before and after maintenance or modifications of the treatment apparatus. This method may be advantageously exercised using the phantom device 6 to give enhanced sensitivity to changes in the properties of the beam or in the alignment of system components. In each irradiation session, the target is prepared in an appropriate manner. Depending on the target used, preparation may, for example, involve filling of water tanks or immobilization of a tissue target. Imaging of the target may be performed in order to plan a beam delivery sequence. The treatment room, target and detector apparatus 3 are then set up for the irradiation session. The radiation beam is delivered according to the prepared sequence, and measurements are acquired using the detector apparatus 3. The detector apparatus 3 may advantageously take as input the parameters of the beam delivery in order to calculate a scheme of movement. Alternatively, the detector apparatus 3 may execute a pre-set scheme of movement which gives sensitivity over the entire volume of the target or the volume of interest.

While the phantom device 6 may be used as a consistent target for enhancing the measurement precision of a constancy check, the detector apparatus 3 may also advantageously be used to assess uncertainties in TPS modelling of other targets, such as tissue samples or animal parts. By way of example, the TPS may be used to construct a model of the target and a treatment plan to be delivered into the target. The simplest possible treatment plan would consist of a single beam spot. Upon or after delivery of a treatment plan, the computing system 4 may produce an image in 1, 2 or 3 dimensions, possibly including time-dependent information (spatiotemporal), of the target activation detected by the detector apparatus 3. The computing system 4 may output at least one distal fall-off coordinate 113 of the measured activation, the distal fall-off coordinate corresponding to a falling edge of the measured activation along the beam axis. This distal fall-off point can be associated with a residual beam energy, corresponding to a cutoff energy for production of a radioactive isotope being imaged by the detector apparatus. The computing system 4 may also output the coordinates of at least one rising edge 114 of measured activation, the rising edge corresponding to the beam entrance point or surface of the irradiated target, so as to provide an independent means of locating the target with respect to the detector apparatus 3.

By making measurements with varying beam energies, the above-described distal fall-off method may be extended to probe the residual beam energy along the axis of the beam. It is advantageous in this case to define a sequence of beam spots which are sufficiently well separated in the target for resolution of individual fall-off points. This may be achieved, for example, by using an ascending sequence of beam energies such that the fall-off point of each spot is resolvable from the residual activity of previous beam spots. The finite decay time of the activated isotopes may also be exploited by delivering beam spots separated by a sufficient time interval to allow spatially-overlapping distributions to substantially decay.

The method may also be used with a field of scanned beam spots, to make an estimate of the residual beam energy at positions over the beam scanning plane.

The previously described methods may be combined to probe the residual beam energy in the target as a function of position in one, two or three dimensions. This may be achieved, for example, by defining a sequence of layered beam spots with defined energies and coordinates in the beam scanning plan, such that each energy layer, or the distal fall-off of each layer, is resolvable for each point in the scanning plane.

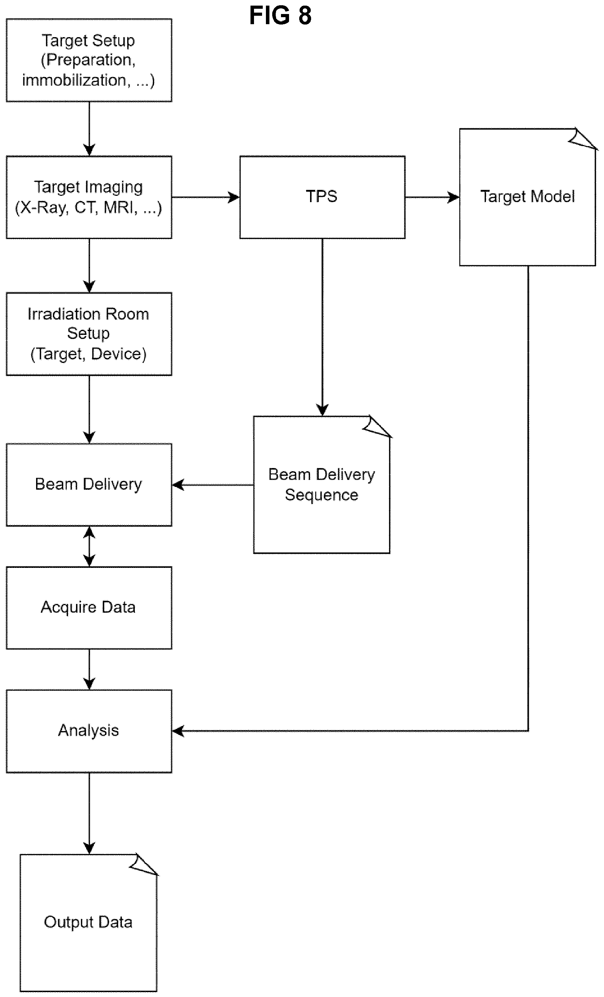

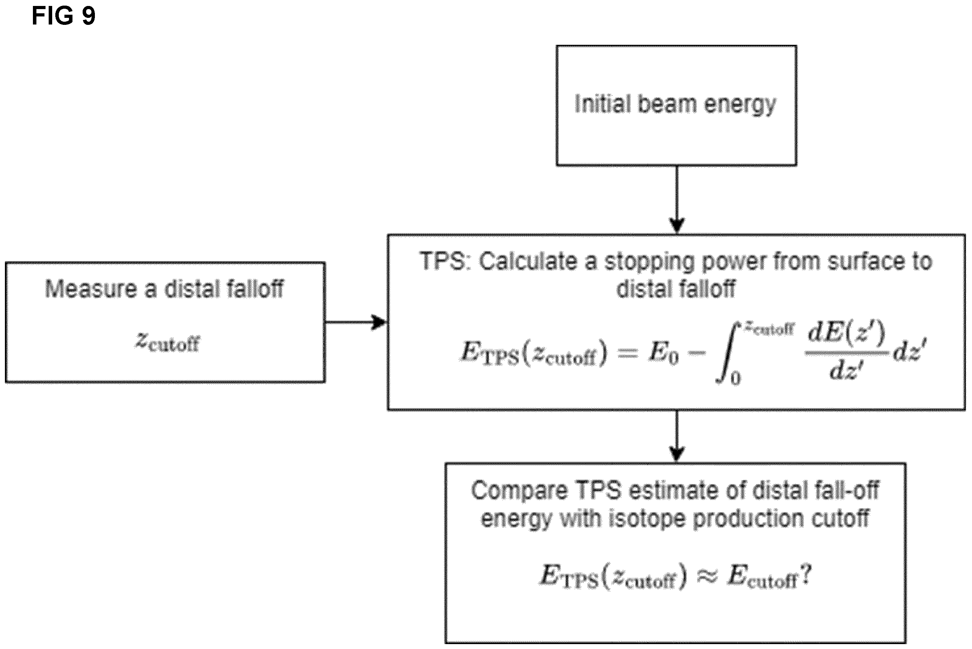

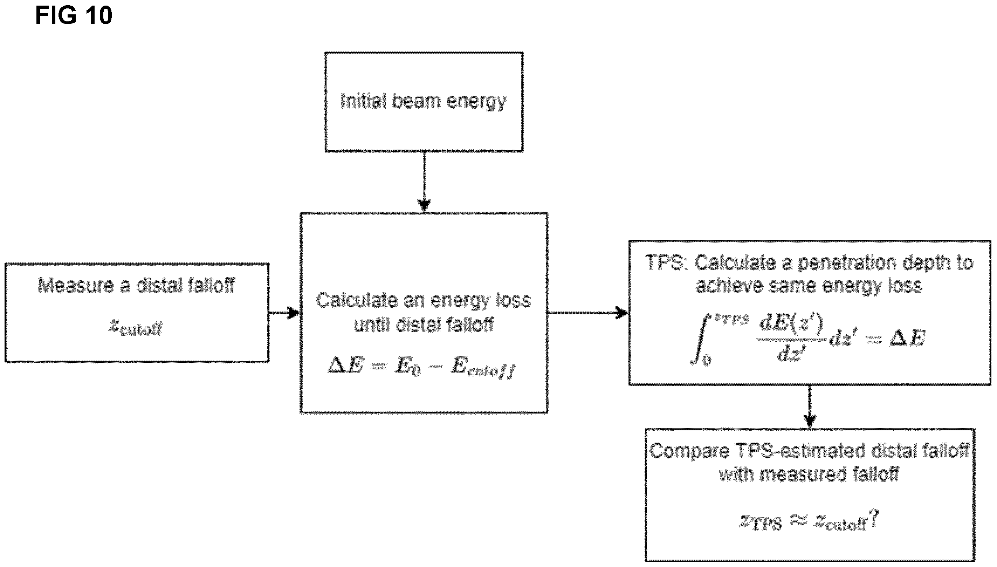

The residual beam energy measurements gained from the methods described previously may be compared to values calculated by existing treatment planning solutions in order to verify the target modelling assumed by these existing solutions. An embodiment of a process for performing an evaluation of the treatment planning system (TPS) is illustrated in Figures 8, 9 and 10. The detector apparatus 3 provides a measurement of the distal fall-off position z_cutoff. The initial energy E_0 is assumed to be known from the beam delivery configuration, while the threshold energy for target activation E_cutoff is assumed to be known from the literature. The TPS provides a calculation of either the residual beam energy at the specified depth z_cutoff, or of the depth to achieve a specified residual beam energy E_cutoff. An evaluation of the consistency of the TPS calculation with the measured result is possible using either quantity. The calculation of these quantities by the TPS relies on its estimate of the stopping power integral, incorporating a model of the beam delivery system, of the target, and of the interaction of beam particles in the target. The comparison of the quantities calculated by the TPS against quantities measured by the detector apparatus 3 is therefore an evaluation of the accuracy of the modelling of these aspects which are relevant to the planning of clinical treatments.

Since the imaged activity endpoint is proximal to the peak of dose deposition and the beam range, the distal activity fall-off is not sensitive to material variations at the distal end of the beam range. It is therefore advantageous to modify the treatment plan to extend the volume which is probed. For example, the treatment plan may be modified to include energies higher than those used in the proposed clinical treatment. In this way the validity of modelling assumptions can be probed over the entire target volume relevant for the planning of dose deposition. Likewise, the number of particles per spot may also be advantageously increased beyond what would be appropriate for clinical practice, thereby increasing the target activation in order to provide sufficient statistics for producing an image of desired quality.

### List of references used

**Treatment apparatus 100**
   Treatment table (patient treatment couch) 101
      Position markers 106
   Robotic arm 102
   Particle radiation device 103
      Accelerator 112
      Gantry 105
         Magnets 109
      Radiation emitter head 104
         Beam monitors 110
      Particle beam 111
   Beam Delivery System 107
   Treatment Planning System 108
**Quality assurance system for radiation therapy 2**
   **Detector apparatus 3**
      Support mechanism 7
         Base 12
         Support arm 14
         Pivot coupling 16
            Pivot base 17
         Slide coupling 19
            Rails
         Detection module support structure 18
      Detection modules 8
   **Computing system 4**
      Fall-off point 113
      Rising edge 114
   **Detector transport apparatus 5**
      Trolley 9
         Guard rail 23
         Height adjustment mechanism 25
         Wheels 27
         Cabinet chamber 34
      Adaptor interface 20
         Base positioning elements 32
         Treatment table fixation elements 35
   **Phantom device 6**
      Body 22
         Material PMMA, ...
      Cavities 24
         Bores 26
         Slots 28
            Transverse 28a
            Axial 28b
         Top face 30
         Side face 31
         Chamfer, rounded corner face 33
   *Axial center axis A*
   *Gantry rotation axis B*

## Claims

1. Quality assurance system for radiation therapy (2) for use in a treatment apparatus (100) including a treatment table (101) and a particle radiation device (103) having a radiation emitter head (104) for particle beam radiation, comprising
- a detector apparatus (3) including at least one detection module (8) configured for the measurement of gamma radiation emitted by a target object subject to a particle radiation beam,
- a support mechanism (7) on which the at least one detection module (8) is mounted, the support mechanism (7) comprising a base (12) configured for removable coupling to a treatment table (101) of the treatment apparatus (100), and at least one detection module support structure (18) pivotally coupled to the base (12), and
- a detector transport apparatus (5) including a trolley (9) having a support surface for removably mounting the detector apparatus thereon, the trolley (9) having ground engaging members allowing displacement of the trolley to and away from a treatment apparatus configured for positioning the detector apparatus (3) for coupling to the treatment apparatus.

2. The system according to claim 1 wherein the detection module support structure (18) has a C-shape with an open section configured for transmission of a particle radiation beam emitted by a radiation emitter (104) of the particle radiation device (103) through the open section.

3. The system according to claim 1 wherein the detection module support structure (18) has at least two open sections configured for transmission of a particle radiation beam emitted by a radiation emitter (104) of the particle radiation device (103) through the open sections.

4. The system according to any preceding claim wherein the ground engaging members are in the form of wheels (27) or rollers

5. The system according to any preceding claim wherein the support mechanism (7) comprises a support arm (14) coupling the detection module support structure (18) to the pivot coupling (16) and positioning the detection module support structure (18) relative to the pivot coupling (16) such that a center axis of the detection module support structure is aligned with the axis of rotation of the pivot coupling.

6. The system according to any preceding claim wherein the support mechanism (7) of the detector apparatus (3) further comprises a slide coupling (19) configured for translation of at least the detection module support structure (18), for instance comprising rails on the base (12) engaging complementary rails or channels on the pivot base (17).

7. The system according to any preceding claim further including an adaptor interface (20) mountable on a treatment table (101) of a treatment apparatus (100), the detector apparatus (3) mountable on the adaptor interface (20) for coupling to the treatment table (101).

8. The system according to any preceding claim further including a phantom device (6) including a body (22) consisting of or comprising a material selected from any one or more of polyethylene (PE), HE Solid Water, Virtual Water, RW1, RW3, polystyrene, Blue Water or Polymethylmethacrylate (PMMA), or other low-Z materials with generally short-lived radioactive isotopes produced by the beam, or isotopes with a high probability of emitting positrons after interaction with a radiation beam (111) such as such as ¹⁸O, ⁶³Cu or ⁶⁸Zn, and one or more cavities (24) formed within the body.

9. The system according to the preceding claim wherein the cavities of the phantom device include bores (26) formed into the material of the body and/or slots (28) which may include transverse slots (28a) and axial slots (28b).

10. The system of any preceding claim further comprising a computing system (4) connectable to the detector apparatus (3) for acquisition of measurement data from the detection modules (8) and for control of the detector apparatus.

11. The system according to the preceding claim where the computing system (4) is configured to output at least one distal fall-off coordinate of the measured activation, the distal fall-off coordinate corresponding to a falling edge of the measured activation along the beam axis.

12. The system according to claim 10 or 11 where the computing system (4) is configured to output the coordinates of at least one rising edge of the measured activation, the rising edge coordinates corresponding to an entrance point of the irradiated target.

13. A treatment apparatus (100) in combination with a quality assurance system for radiation therapy according to any preceding claim, wherein in use the detector apparatus (3) is mounted on a treatment table (101) of the treatment apparatus and a radiation emitter head (104) of the treatment apparatus is positioned for transmission of a particle radiation beam through an opening in the detection module support structure (18), the position of the detection module support structure adjusted for alignment with the radiation emitter head (104) through the opening in the support structure (18).

14. The combination according to the preceding claim wherein the detection module support structure is dynamically coupled to the base (12) and configured to rotate in a synchronized manner with the radiation emitter head (104) of the treatment apparatus (100).

15. The combination of either of the two directly preceding claims wherein the treatment apparatus comprises a treatment planning module and a beam delivery system and wherein the computing system (4) of the quality assurance system for radiation therapy (2) according to any of claims 10-12 is connected to at least one of the treatment planning system module of the treatment apparatus (100) and the beam delivery system for receiving information on the target, the treatment plan, the irradiation progress and log files from the irradiation session.

## Patentansprüche

1. Qualitätssicherungssystem für Strahlentherapie (2) zur Verwendung in einer Behandlungseinrichtung (100), die einen Behandlungstisch (101) und eine Partikelstrahlungsvorrichtung (103) umfasst, die einen Strahlungsemitterkopf (104) zur Partikelstrahlstrahlung aufweist, umfassend:
- eine Detektoreinrichtung (3), die mindestens ein Detektionsmodul (8) umfasst, das zur Messung von Gammastrahlung ausgestaltet ist, die von einer Zielobjektperson zu einem Partikelstrahlungsstrahl emittiert wird,
- einen Stützmechanismus (7), an dem das mindestens eine Detektionsmodul (8) montiert ist, wobei der Stützmechanismus (7) eine Basis (12), die zum abnehmbaren Koppeln an einen Behandlungstisch (101) der Behandlungseinrichtung (100) ausgestaltet ist, und mindestens eine Detektionsmodul-Stützstruktur (18) umfasst, die schwenkbar an die Basis (12) gekoppelt ist, und
- eine Detektortransporteinrichtung (5), die einen Transportwagen (9) umfasst, der eine Stützoberfläche zum abnehmbaren Montieren der Detektoreinrichtung daran aufweist, wobei der Transportwagen (9) Bodeneingriffselemente aufweist, die Verlagerung des Transportwagens zu einer Behandlungseinrichtung und davon weg erlauben und zum Positionieren der Detektoreinrichtung (3) zum Koppeln an die Behandlungseinrichtung ausgestaltet sind.

2. System nach Anspruch 1, wobei die Detektionsmodul-Stützstruktur (18) eine C-Form mit einer offenen Sektion aufweist, die zum Durchlassen eines von einem Strahlungsemitter (104) der Partikelstrahlungsvorrichtung (103) emittierten Partikelstrahlungsstrahls durch die offene Sektion ausgestaltet ist.

3. System nach Anspruch 1, wobei die Detektionsmodul-Stützstruktur (18) mindestens zwei offene Sektionen aufweist, die zum Durchlassen eines von einem Strahlungsemitter (104) der Partikelstrahlungsvorrichtung (103) emittierten Partikelstrahlungsstrahls durch die offenen Sektionen ausgestaltet ist.

4. System nach einem der vorhergehenden Ansprüche, wobei die Bodeneingriffselemente in der Form von Rädern (27) oder Rollen vorliegen.

5. System nach einem der vorhergehenden Ansprüche, wobei der Stützmechanismus (7) einen Stützarm (14) umfasst, der die Detektionsmodul-Stützstruktur (18) mit der Schwenkkupplung (16) koppelt und die Detektionsmodul-Stützstruktur (18) derart in Bezug auf die Schwenkkupplung (16) positioniert, dass eine Mittelachse der Detektionsmodul-Stützstruktur mit der Drehachse der Schwenkkupplung ausgerichtet ist.

6. System nach einem der vorhergehenden Ansprüche, wobei der Stützmechanismus (7) der Detektoreinrichtung (3) ferner eine Schiebekupplung (19) umfasst, die zur Translation von mindestens der Detektionsmodul-Stützstruktur (18), ausgestaltet ist und zum Beispiel Schienen auf der Basis (12) umfasst, die in ergänzende Schienen oder Kanäle der Schwenkbasis (17) eingreifen.

7. System nach einem der vorhergehenden Ansprüche, das ferner eine Adapterschnittstelle (20) umfasst, die an einem Behandlungstisch (101) einer Behandlungseinrichtung (100) montierbar ist, wobei die Detektoreinrichtung (3) an der Adapterschnittstelle (20) zum Koppeln an den Behandlungstisch (101) montierbar ist.

8. System nach einem der vorhergehenden Ansprüche, das ferner eine Phantomvorrichtung (6) umfasst, die einen Körper (22), der aus einem Material besteht, das ausgewählt ist aus einem oder mehreren von Polyethylen (PE), HE-Festwasser, virtuellem Wasser, RW1, RW3, Polystyrol, blauem Wasser oder Polymethylmethacrylat (PMMA) oder anderen niedrig-Z-Materialien mit allgemein kurzlebigen radioaktiven Isotopen, die durch den Strahl erzeugt werden, oder Isotopen mit einer hohen Wahrscheinlichkeit der Emission von Positronen nach der Interaktion mit einem Strahlungsstrahl (111), wie beispielsweise ¹⁸O, ⁶³Cu oder ⁶⁸Zn, oder dieses umfasst, und einen oder mehrere Hohlräume (24) umfasst, die innerhalb des Körpers gebildet sind.

9. System nach dem vorhergehenden Anspruch, wobei die Hohlräume der Phantomvorrichtung Bohrungen (26), die in das Material des Körpers gebildet sind, und/oder Schlitze (28) umfassen, die Querschlitze (28a) und axiale Schlitze (28b) umfassen können.

10. System nach einem der vorhergehenden Ansprüche, das ferner ein Rechensystem (4) umfasst, das mit der Detektorvorrichtung (3) zur Erfassung von Messdaten von den Detektionsmodulen (8) und zur Steuerung der Detektoreinrichtung verbindbar ist.

11. System nach dem vorhergehenden Anspruch, wobei das Rechensystem (4) dazu ausgestaltet ist, mindestens eine distale Abfallkoordinate der gemessenen Aktivierung auszugeben, wobei die distale Abfallkoordinate einer abfallenden Flanke der gemessenen Aktivierung entlang der Stahlachse entspricht.

12. System nach Anspruch 10 oder 11, wobei das Rechensystem (4) dazu ausgestaltet ist, die Koordinaten von mindestens einer ansteigenden Flanke der gemessenen Aktivierung auszugeben, wobei die ansteigenden Flankenkoordinaten einem Eintrittspunkt des bestrahlten Ziels entsprechen.

13. Behandlungseinrichtung (100) in Kombination mit einem Qualitätssicherungssystem für Strahlentherapie nach einem der vorhergehenden Ansprüche, wobei die Detektoreinrichtung (3) bei der Verwendung auf einem Behandlungstisch (101) der Behandlungseinrichtung montiert ist und ein Strahlungsemitterkopf (104) der Behandlungseinrichtung zum Durchlassen eines Partikelstrahlungsstrahls durch eine Öffnung in der Detektionsmodul-Stützstruktur (18) positioniert ist, wobei die Position der Detektionsmodul-Stützstruktur zur Ausrichtung mit dem Strahlungsemitterkopf (104) durch die Öffnung in der Stützstruktur (18) angepasst wird.

14. Kombination nach dem vorhergehenden Anspruch, wobei die Detektionsmodul-Stützstruktur dynamisch an die Basis (12) gekoppelt ist und dazu ausgestaltet ist, sich auf eine synchronisierte Weise mit dem Strahlungsemitterkopf (104) der Behandlungseinrichtung (100) zu drehen.

15. Kombination nach einem der zwei direkt vorhergehenden Ansprüche, wobei die Behandlungseinrichtung ein Behandlungsplanungsmodul und ein Strahlabgabesystem umfasst und wobei das Rechensystem (4) des Qualitätssicherungssystems für Strahlentherapie (2) nach einem der Ansprüche 10 bis 12 mit mindestens einem von dem Behandlungsplanungssystemmodul der Behandlungseinrichtung (100) und dem Strahlenabgabesystem zum Empfangen von Informationen über das Ziel, den Behandlungsplan, den Bestrahlungsfortschritt und Protokolldateien von der Bestrahlungssitzung verbunden ist.

## Revendications

1. Système d'assurance qualité pour radiothérapie (2) destiné à être utilisé dans un appareil de traitement (100) comportant une table de traitement (101) et un dispositif de rayonnement de particules (103) doté d'une tête d'émission de rayonnement (104) pour un rayonnement de faisceau de particules, comprenant
- un appareil détecteur (3) comportant au moins un module de détection (8) configuré pour la mesure du rayonnement gamma émis par un objet cible soumis à un faisceau de rayonnement de particules,
- un mécanisme de support (7) sur lequel l'au moins un module de détection (8) est monté, le mécanisme de support (7) comprenant une base (12) configurée pour être couplée de manière amovible à une table de traitement (101) de l'appareil de traitement (100), et au moins une structure de support de module de détection (18) couplée de manière pivotante à la base (12), et
- un appareil de transport de détecteur (5) comportant un chariot (9) ayant une surface de support pour monter de manière amovible l'appareil détecteur là-dessus, le chariot (9) ayant des éléments de mise en prise avec le sol permettant de rapprocher et d'éloigner le chariot d'un appareil de traitement, configuré pour positionner l'appareil détecteur (3) pour le coupler à l'appareil de traitement.

2. Système selon la revendication 1, dans lequel la structure de support de module de détection (18) a une forme en C avec une section ouverte configurée pour la transmission d'un faisceau de rayonnement de particules émis par un émetteur de rayonnement (104) du dispositif de rayonnement de particules (103) à travers la section ouverte.

3. Système selon la revendication 1, dans lequel la structure de support de module de détection (18) a au moins deux sections ouvertes configurées pour la transmission d'un faisceau de rayonnement de particules émis par un émetteur de rayonnement (104) du dispositif de rayonnement de particules (103) à travers les sections ouvertes.

4. Système selon l'une des revendications précédentes, dans lequel les éléments de mise en prise avec le sol sont sous la forme de roues (27) ou de rouleaux.

5. Système selon l'une des revendications précédentes, dans lequel le mécanisme de support (7) comprend un bras de support (14) couplant la structure de support de module de détection (18) au couplage à pivot (16) et positionnant la structure de support de module de détection (18) par rapport au couplage à pivot (16) de sorte qu'un axe central de la structure de support de module de détection soit aligné avec l'axe de rotation du couplage à pivot.

6. Système selon l'une des revendications précédentes, dans lequel le mécanisme de support (7) de l'appareil détecteur (3) comprend en outre un couplage à glissière (19) configuré pour la translation de l'au moins une structure de support de module de détection (18), par exemple comprenant des rails sur la base (12) s'engageant dans des rails ou des canaux complémentaires sur la base de pivot (17).

7. Système selon l'une des revendications précédentes comportant en outre une interface d'adapteur (20) pouvant être montée sur une table de traitement (101) d'un appareil de traitement (100), l'appareil détecteur (3) pouvant être monté sur l'interface d'adapteur (20) pour le coupler à la table de traitement (101).

8. Système selon l'une des revendications précédentes, comportant en outre un dispositif fantôme (6) comportant un corps (22) constitué ou comprenant un matériau choisi parmi un ou plusieurs parmi le polyéthylène (PE), l'eau solide HE, l'eau virtuelle, RW1, RW3, le polystyrène, l'eau bleue ou le polyméthylméthacrylate (PMMA), ou d'autres matériaux à faible Z avec des isotopes radioactifs généralement à courte durée de vie produits par le faisceau, ou des isotopes ayant une forte probabilité d'émettre des positrons après interaction avec un faisceau de rayonnement (111) tels que ¹⁸O, ⁶³Cu ou ⁶⁸Zn, et une ou plusieurs cavités (24) formées à l'intérieur du corps.

9. Système selon la revendication précédente dans lequel les cavités du dispositif fantôme comportent des alésages (26) formés dans le matériau du corps et/ou des fentes (28) qui peuvent comporter des fentes transversales (28a) et des fentes axiales (28b).

10. Système selon l'une des revendications précédentes, comprenant en outre un système informatique (4) pouvant être connecté à l'appareil détecteur (3) pour l'acquisition de données de mesure à partir de modules de détection (8) et pour commander l'appareil détecteur.

11. Système selon la revendication précédente où le système informatique (4) est configuré pour délivrer au moins une coordonnée de chute distale de l'activation mesurée, la coordonnée de chute distale correspondant à un front descendant de l'activation mesurée le long de l'axe de faisceau.

12. Système selon la revendication 10 ou 11, où le système informatique (4) est configuré pour fournir les coordonnées d'au moins un front montant de l'activation mesurée, les coordonnées de front montant correspondant à un point d'entrée de la cible irradiée.

13. Appareil de traitement (100) en combinaison avec un système d'assurance qualité selon l'une des revendications précédentes, dans lequel, en cours d'utilisation, l'appareil détecteur (3) est monté sur une table de traitement (101) de l'appareil de traitement, et une tête d'émission de rayonnement (104) de l'appareil de traitement est positionnée pour la transmission d'un faisceau de rayonnement de particules à travers une ouverture dans la structure de support de module de détection (18), la position de la structure de support de module de détection étant ajustée pour l'alignement avec la tête d'émission de rayonnement (104) à travers l'ouverture dans la structure de support (18).

14. Combinaison selon la revendication précédente dans laquelle la structure de support de module de détection est couplée dynamiquement à la base (12) et configurée pour tourner de manière synchronisée avec la tête d'émission de rayonnement (104) de l'appareil de traitement (100).

15. Combinaison de l'une des deux revendications directement précédentes, dans laquelle l'appareil de traitement comprend un module de planification de traitement et un système d'émission de faisceaux, et dans laquelle le système informatique (4) du système d'assurance qualité pour radiothérapie (2) selon l'une des revendications 10 à 12 est connecté à au moins un parmi le module de système de planification de traitement de l'appareil de traitement (100) et le système d'émission de faisceaux pour recevoir des informations sur la cible, le plan de traitement, la progression de l'irradiation et les fichiers journaux de la session d'irradiation.
